# EUROPEAN PATENT SPECIFICATION

(11) **EP 2 675 797 B1**
(45) Date of publication and mention of the grant of the patent: **16.03.2016**
(21) Application number: 11845221.8
(22) Date of filing: 23.11.2011
(51) Int. Cl.: C07D 295/084, C07C 209/22

(54) **PROCESSES FOR THE PREPARATION OF ENAMINES**
VERFAHREN ZUR HERSTELLUNG VON ENAMINEN
PROCESSUS DE PRÉPARATION D'ÉNAMINES

(30) Priority: 03.12.2010 US 419296 P
(43) Date of publication of application: 25.12.2013
(73) Proprietor: Dow AgroSciences LLC, Indianapolis, IN 46268 (US)
(72) Inventor: BLAND, Douglas C., Midland, MI 48642 (US); TOYZAN, Todd William, Freeland, MI 48623 (US)
(74) Representative: Weickmann & Weickmann
(86) International application number: PCT/US2011/061981
(87) International publication number: WO 2012/074858

(56) References cited:
- WO-A2-2009/007460
- US-A- 3 865 791
- US-A1- 2010 004 457
- US-B1- 6 353 118

## Description

### CROSS-REFERENCE TO RELATED APPLICATIONS

This Application claims priority from U.S. provisional application 61/419,296 filed on December 3, 2010.

### FIELD OF THE INVENTION

The invention disclosed in this document is related to the field of processes for the preparation of enamines.

### BACKGROUND OF THE INVENTION

Enamines are very useful molecules. They have been used in a wide variety of reactions such as, for example, electrophilic substitution and addition, oxidation and reduction, and cycloaddition (J. Kang, Y. R. Cho, and J. H. Lee, Bull. Korean Chem Soc. Vol. 13, No.2, 1992).

An early method for preparing enamines involved the condensation of aldehydes and ketones with secondary amines (C. Mannich and H. Davidsen, Ber., 69, 2106 (1936)). Mannich and Davidsen discovered that the condensation reaction of an aldehyde with a secondary amine could be conducted at temperatures near 0 °C in the presence of potassium carbonate (K₂CO₃), but however, the condensation reaction of a ketone with a secondary amine required calcium oxide (CaO) and elevated temperatures. Later, Herr and Heyl discovered that this type of condensation reaction could be improved by removing water (H₂O) during an azeotropic distillation with benzene (M.E. Herr and F. W. Heyl, J. Am. Chem. Soc., 74, 3627 (1952); F. W. Heyl and M.E. Herr , J. Am. Chem. Soc., 75, 1918 (1953); M.E. Herr and F. W. Heyl, J. Am. Chem. Soc., 75, 5927 (1953); F. W. Heyl and M.E. Herr , J. Am. Chem. Soc., 77, 488 (1955)). Since these publications a number of modifications have been disclosed. Usually, these modifications are based on using dehydration reagents such as K₂CO₃, CaO, *p*-toluenesulfonic acid (TsOH), boron trifluoride diethyl etherate (BF₃-OEt₂), acetic acid (AcOH), magnesium sulfate (MgSO₄), calcium hydride (CaH₂), titanium tetrachloride (TiCl₄), and molecular sieves (see J. Kang above). Other modifications deal with chemically converting water to something else during the condensation reaction (see J. Kang above). An extensive summary of the vast number of methods to prepare enamines is discussed in "ENAMINES, Synthesis, Structure, and Reactions, 2nd Edition, Edited by A. G. Cook, Chap. 2, (1988). Specific examples of processes to prepare enamines can be found in the following:
U.S. Patent 3,074,940 which discloses that certain aldehydes form azeotropes with water which can be used to remove the reaction water formed during certain enamine condensation reactions;
U.S. Patent 3,530,120 which discloses conducting certain enamine condensation reactions in an inert atmosphere with certain arsine molecules;
U.S. Patent 5,247,091 which discloses conducting certain enamine condensation reactions in an aqueous media;
S. Kaiser, S. P. Smidt, and A. Pfaltz, Angew. Int. Ed. 2006, 45, 5194-5197 - See Supporting information pages 10-11; and

WO 2009/007460 A2, see page 13, example 1.a.

Enamines such as 1-(3-thiobut-1-enyl)pyrrolidine are useful intermediates for the preparation of certain new insecticides (see, for example, U.S. Patent Publications 2005/0228027 and 2007/0203191). Current known processes to make such thioenamines are not efficient in producing such enamines due to a variety of reasons -- there are problems in preventing thermal degradation of the thioenamine, and while using potassium carbonate is an effective desiccant, it is problematic to filter such desiccant during larger than lab-scale production. Thus, a process is needed to remove water during these types of condensation reactions without using solid desiccants, or using temperature conditions that promote the thermal degradation of such enamines.

### DETAILED DESCRIPTION OF THE INVENTION

In general, the processes disclosed in this document can be illustrated as in Scheme 1.

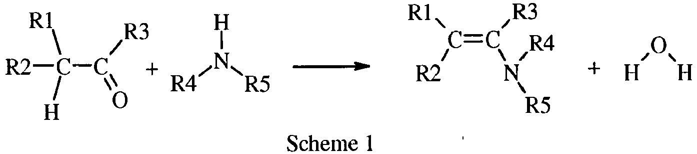

In general, the invention is a process comprising:
(A) contacting, in a reaction zone, a first mixture with a second mixture
   (1) wherein said first mixture comprises a carbonyl (i.e. an aldehyde or a ketone) having the following formula 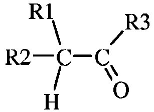
      (a) wherein R1 and R2 is each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, each of which is independently substituted with one or more S-R6 wherein each R6 is independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, and
      (b) wherein R3 is selected from H, C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or wherein said carbonyl is 3-methylsulfanyl-butyraldehyde
   (2) wherein said second mixture comprises a non-polar-high-boiling-point solvent and an amine having the following formula 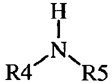 wherein R4 and R5 are each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or R4 and R5 taken together with N represent a 5- or 6-membered saturated or unsaturated ring;
(B) reacting in said reaction zone said amine and said carbonyl to produce an enamine and H₂O, wherein reacting is conducted under distillation conditions comprising
   (1) a pressure from about 100 Pascals (Pa) to about 120,000 Pa, and
   (2) a temperature below about, but preferably below, the thermal decomposition temperature of said enanine during said reacting; and
(C) removing a vapor phase comprising said non-polar-high-boiling-point-solvent, amine, and H₂O; and
(D) condensing said vapor phase from step (C) to produce a condensate; and
(E) contacting said condensate from step (D) with a recovery mixture comprising H₂O and an amine-rejecting agent selected from the group consisting of sodium hydroxide and a brine solution to produce a separate mixture comprising said amine; and
(F) optionally, returning said amine from step (E) back to said reaction zone.

Approximately equimolar quantities of said amine and said carbonyl can be used in the process, although excesses of one or the other may be employed. The molar ratio of amine to carbonyl can be from about 0.9 to about 1.2, however, a slight molar excess of amine to carbonyl is preferred, such as, for example, a molar ratio greater than 1 but less than about 1.1.

The reaction is conducted in the presence of a non-polar-high-boiling-point-solvent such as, hydrocarbon solvents, most preferably aromatic hydrocarbon solvents such as, for example, benzene, toluene, or xylene. Currently, toluene is a preferred solvent.

In another embodiment of this invention said reacting is conducted under distillation conditions comprising a pressure from about 1000 Pa to about 60,000 Pa and a temperature from about 10 ° C to about 80 ° C.

In another embodiment of this invention said reacting is conducted under distillation conditions comprising a pressure from about 2500 Pa to about 30,000 Pa and a temperature from about 20 °C to about 70 °C.

In another embodiment of this invention said reacting is conducted under distillation conditions comprising a pressure from about 5000 Pa to about 15,000 Pa and a temperature from about 25 °C to about 65 °C. In another embodiment of this invention when producing 1-(3-methylsulfanyl-but-l-enyl)-pyrrolidine a temperature below about the thermal decomposition temperature of 1-(3-methylsulfanyl-but-1-enyl)-pyrrolidine during said reacting is preferred.

It is preferred in such processes that the condensation reaction be conducted under azeotropic conditions so that as much water can be removed as desired. It is also preferred if no desiccants be used to remove water.

In another embodiment of this invention, R1 and R2 are independently C₁-C₈ alkyl, C₃-C₈ cycloalkyl, each of which is independently substituted with one or more S-R6 wherein each R6 is independently selected from C₁-C₈ alkyl.

In another embodiment of this invention, R3 is H.

In another embodiment of this invention, R4 and R5 are each independently selected from C₁-C₈ alkyl, and C₃-C₈ cycloalkyl. In another embodiment of this invention R4 and R5 taken together with N represent a 5- or 6-membered saturated or unsaturated ring.

In another embodiment of this invention, said first mixture comprises pyrrolidine and said second mixture comprises 3-methylsulfanyl-butyraldehyde. In another embodiment of this invention, said enamine is 1-(3-methylsulfanyl-but-1-enyl)-pyrrolidine.

In another embodiment of this invention, the first mixture and second mixture can be contacted in the reaction zone simultaneously as they are added.

Said recovery mixture comprises an amine rejecting agent. An amine rejecting agent is an agent that is ionic and that dissolves in water readily, such as sodium hydroxide and brine solutions. Preferably the amine rejecting agent is concentrated in H₂O to greater than 25 weight percent sodium hydroxide, such as about 25 to about 50 weight percent sodium hydroxide.

### EXAMPLES

The examples are for illustration purposes and are not to be construed as limiting the invention disclosed in this document to only the embodiments disclosed in these examples.

### Comparative Example Preparation of 1-(3- methylthiobut-1-enyl)pyrrolidine.

A three-neck 250 mL round bottom flask equipped with a short path distillation head was connected to a receiver flask containing a dry-ice acetone condenser. To this reaction vessel was charged 19.8 g (0.28 mol) of pyrrolidine followed by 70 mL of toluene. The mixture was cooled in an ice-water bath until the internal reaction pot temperature was about 3 °C. Then vacuum (about 3300 Pa) was applied to the system and then 94.4 g (0.14 mol) of 3-methylthiobutanal as a 17.5 wt% solution in toluene was continuously added to the reaction mixture via syringe over a one hour (h) period. The internal reaction temperature rose from 3 °C up to 18 °C during addition of the aldehyde solution. Distillate was also collected during aldehyde addition. Upon completing addition of the 3-methylthiobutanal solution, the distillation was continued for an additional 50 minutes (min) until the internal pot temperature reached 26 °C. At this time, the vacuum was adjusted to about 2400 Pa and the distillation was continued for an additional 2.0 min until the internal pot temperature reached 24 °C. The distillation was stopped and the reaction vessel was padded with nitrogen. The reactive distillation bottoms were isolated to give 74.91 g of 1-(3-methylthiobut-1-enyl)pyrrolidine was a 28 wt% yellow solution in toluene. Proton (¹H) NMR spectroscopic assay of the solution mixture (using benzyl acetate as the internal standard) indicated a 84% in-pot yield.

### Example #1 Preparation of 1-(3-methylthiobut-1-enyl)pyrrolidine.

A three-neck 250 mL round bottom flask was equipped with a Dean-Stark trap, addition funnel, and magnetic stir bar. On top of the Dean Stark trap was stacked a water cooled condenser followed by a dry-ice acetone condenser. To the Dean-Stark trap collection reservoir was charged 11 g of 50 wt% aqueous sodium hydroxide and this collection reservoir was cooled in an ice-water bath. To the 250 mL reaction vessel was charged 10.95 g (0.15 mol) of pyrrolidine followed by 70 mL of toluene. A vacuum (about 6600 Pa) was applied to the system and toluene was allowed to collect into the Dean-Stark trap collection reservoir. Once the reflux return from the Dean Stark trap to the reaction pot had been established, a 94.4 g (0.14 mol) of 3- methylthiobutanal as a 17.5 wt% in toluene solution was continuously added through the addition funnel over a 1 h and 15 min period. The internal reaction temperature was maintained below 24 °C during the aldehyde addition. Upon completing addition of the 3-methylthiobutanal, the distillation was stopped and the Dean-Stark trap reservoir was drained. The Dean-Stark trap reservoir was then filled with 2 mL of distilled water and the distillation was continued at about a 6600 Pa vacuum for 70 min until the internal pot temperature reached 30 °C. At this time, the distillation was halted and the Dean-Stark trap reservoir was drained. The Dean-Stark trap was then replaced with a short path distillation head and the distillation was continued at about 6600 Pa for an additional 30 min until the pot temperature reached 33 °C. The vacuum was adjusted to about a 2400 Pa and the distillation was continued until the pot temperature reached 21 °C at which time the distillation was halted and the reaction vessel was padded with nitrogen. A total of 59 g of distillate was collected overhead. The reactive distillation bottoms were isolated to give 72.26 g of 1-(3-methylthiobut-1-enyl)pyrrolidine was a 27.6 wt% yellow solution in toluene. Proton NMR spectroscopic assay of the solution mixture (using benzyl acetate as the internal standard) indicated a 83% in-pot yield.

In the comparative example about twice as much amine had to be used to obtain good yields as opposed to Example 1.

## Claims

1. A process comprising:
(A) contacting, in a reaction zone, a first mixture with a second mixture
(1) wherein said first mixture comprises a carbonyl, an aldehyde or a ketone, having the following formula 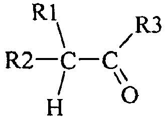
(a) wherein R1 and R2 is each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, each of which is independently substituted with one or more S-R6 wherein each R6 is independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, and
(b) wherein R3 is selected from H, C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or wherein said carbonyl is 3-methylsulfanyl-butyraldehyde, and
(2) wherein said second mixture comprises a non-polar-high-boiling-point solvent and an amine having the following formula 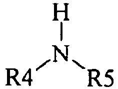 wherein R4 and R5 are each independently selected from C₁-C₈ alkyl, C₃-C₈ cycloalkyl, C₂-C₈ alkoxyalkyl, C₇-C₁₂ arylalkyl, C₂-C₈ alkylaminoalkyl, aryl, and heteroaryl, or R4 and R5 taken together with N represent a 5- or 6-membered saturated or unsaturated ring;
(B) reacting in said reaction zone said amine and said carbonyl to produce an enamine and H₂O, wherein reacting is conducted under distillation conditions comprising
(1) a pressure from about 100 Pascals (Pa) to about 120,000 Pa, and
(2) a temperature below about, but preferably below, the thermal decomposition temperature of said enamine during said reacting; and
(C) removing a vapor phase comprising said non-polar-high-boiling-point-solvent, amine, and H₂O; and
(D) condensing said vapor phase from step (C) to produce a condensate; and
(E) contacting said condensate from step (D) with a recovery mixture comprising H₂O and an amine-rejecting agent selected from the group consisting of sodium hydroxide and a brine solution to produce a separate mixture comprising said amine; and
(F) optionally, returning said amine from step (E) back to said reaction zone.

2. A process according to claim 1 wherein approximately equimolar quantities of said amine and said carbonyl are used.

3. A process according to claim 1 wherein the molar ratio of amine to carbonyl is from about 0.9 to about 1.2 or wherein the molar ratio of amine to carbonyl is greater than 1 but less than about 1.1.

4. A process according to claim 1 wherein said non-polar-high-boiling-point-solvent is an aromatic hydrocarbon solvent or wherein said non-polar-high-boiling-point-solvent is benzene or wherein said non-polar-high-boiling-point-solvent is toluene or wherein said non-polar-high-boiling-point-solvent is xylene.

5. A process according to claim 1 wherein said reacting is conducted under distillation conditions comprising a pressure from about 1000 Pa to about 60,000 Pa and a temperature from about 10°C to about 80°C or wherein said reacting is conducted under distillation conditions comprising a pressure from about 2500 Pa to about 30,000 Pa and a temperature from about 20°C to about 70°C or wherein said reacting is conducted under distillation conditions comprising a pressure from about 5000 Pa to about 15,000 Pa and a temperature from about 25°C to about 65°C.

6. A process according to claim 1 wherein said process is producing 1-(3-methylsulfanyl-but-1-enyl)-pyrrolidine at a temperature below about the thermal decomposition temperature of said 1-(3-methylsulfanyl-but-1-enyl)-pyrrolidine during said reacting.

7. A process according to claim 1 wherein said condensation reaction is conducted under azeotropic conditions.

8. A process according to claim 1 wherein a desiccant is not used to remove water.

9. A process according to claim 1 wherein R1 and R2 are independently C₁-C₈ alkyl, C₃-C₈ cycloalkyl, each of which is independently substituted with one or more S-R6 wherein each R6 is independently selected from C₁-C₈ alkyl.

10. A process according to claim 1 wherein R3 is H.

11. A process according to claim 1 wherein R4 and R5 are each independently selected from C₁-C₈ alkyl, and C₃-C₈ cycloalkyl or wherein R4 and R5 taken together with N represent a 5- or 6-membered saturated or unsaturated ring.

12. A process according to claim 1 wherein said first mixture comprises 3-methylsulfanyl-butyraldehyde and said second mixture comprises pyrrolidine.

13. A process according to claim 1 wherein said enamine is 1-(3-methylsulfanyl-but-1-enyl)-pyrrolidine.

14. A process according to claim 1 wherein the first mixture and second mixture can be contacted in the reaction zone simultaneously as they are added.

15. A process according to claim 1 wherein said recovery mixture comprises an amine rejecting agent where said amine rejecting agent is sodium hydroxide or wherein said recovery mixture comprises an amine rejecting agent where said amine rejecting agent is sodium hydroxide and said sodium hydroxide is concentrated in H₂O from about 25 to about 50 weight percent sodium hydroxide or wherein said recovery mixture comprises an amine rejecting agent where said amine rejecting agent is a brine solution.

## Patentansprüche

1. Verfahren, umfassend:
(A) In-Kontakt-bringen, in einer Reaktionszone, eines ersten Gemischs mit einem zweiten Gemisch
(1) worin das erste Gemisch ein Carbonyl, ein Aldehyd oder ein Keton mit der folgenden Formel 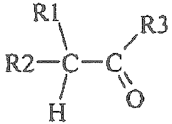 umfasst,
(a) worin R1 und R2 jeweils unabhängig ausgewählt sind aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl, wobei jedes davon unabhängig substituiert ist mit einem oder mehreren S-R6, worin jedes R6 unabhängig ausgewählt ist aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl, und
(b) worin R3 ausgewählt ist aus H, C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl, oder worin das Carbonyl 3-Methylsulfanyl-butyraldehyd ist, und
(2) worin das zweite Gemisch ein hochsiedendes nichtpolares Lösungsmittel und ein Amin mit der folgenden Formel 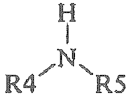 umfasst,
worin R4 und R5 jeweils unabhängig ausgewählt sind aus C₁-C₈-Alkyl, C₃-C₈-Cycloalkyl, C₂-C₈-Alkoxyalkyl, C₇-C₁₂-Arylalkyl, C₂-C₈-Alkylaminoalkyl, Aryl und Heteroaryl, oder R4 und R5 zusammengenommen mit N einen 5- oder 6-gliedrigen gesättigten oder ungesättigten Ring bedeuten;
(B) Umsetzen des Amins und des Carbonyls in der Reaktionszone, um ein Enamin und H₂O zu erzeugen, worin das Umsetzen unter Destillationsbedingungen durchgeführt wird, umfassend
(1) einen Druck von etwa 100 Pascal (Pa) bis etwa 120000 Pa und
(2) eine Temperatur unter etwa, jedoch bevorzugt unter, der thermischen Zersetzungstemperatur des Amins während des Umsetzens; und
(C) Entfernen einer Dampfphase, die das hochsiedende nichtpolare Lösungsmittel, Amin und H₂O umfasst; und
(D) Kondensieren der Dampfphase aus Schritt (C), um ein Kondensat zu erzeugen; und
(E) In-Kontakt-bringen des Kondensats aus Schritt (D) mit einem Rückgewinnungsgemisch, umfassend H₂O und ein AminAbscheidungsmittel, ausgewählt aus der Gruppe, bestehend aus Natriumhydroxid und einer Salzlösung, um ein getrenntes Gemisch zu erzeugen, das das Amin umfasst; und
(F) optional Rückführen des Amins aus Schritt (E) zurück in die Reaktionszone.

2. Verfahren nach Anspruch 1, worin ungefähr äquimolare Mengen des Amins und des Carbonyls verwendet werden.

3. Verfahren nach Anspruch 1, worin das molare Verhältnis von Amin zu Carbonyl von etwa 0,9 bis etwa 1,2 ist oder worin das molare Verhältnis von Amin zu Carbonyl größer als 1 aber kleiner als etwa 1,1 ist.

4. Verfahren nach Anspruch 1, worin das hochsiedende nichtpolare Lösungsmittel ein aromatisches Kohlenwasserstofflösungsmittel ist oder worin das hochsiedende nichtpolare Lösungsmittel Benzol ist oder worin das hochsiedende nichtpolare Lösungsmittel Toluol ist oder worin das hochsiedende nichtpolare Lösungsmittel Xylol ist.

5. Verfahren nach Anspruch 1, worin das Umsetzen unter Destillationsbedingungen durchgeführt wird, umfassend einen Druck von etwa 1000 Pa bis etwa 60000 Pa und eine Temperatur von etwa 10 °C bis etwa 80 °C, oder worin das Umsetzen unter Destillationsbedingungen durchgeführt wird, umfassend einen Druck von etwa 2500 Pa bis etwa 30000 Pa und eine Temperatur von etwa 20 °C bis etwa 70 °C, oder worin das Umsetzen unter Destillationsbedingungen durchgeführt wird, umfassend einen Druck von etwa 5000 Pa bis etwa 15000 Pa und eine Temperatur von etwa 25 °C bis etwa 65 °C.

6. Verfahren nach Anspruch 1, worin das Verfahren 1-(3-Methylsulfanyl-but-1-enyl)-pyrrolidin bei einer Temperatur unter etwa der thermischen Zersetzungstemperatur des 1-(3-Methylsulfanyl-but-1-enyl)-pyrrolidins während der Reaktion erzeugt.

7. Verfahren nach Anspruch 1, worin die Kondensationsreaktion unter azeotropen Bedingungen durchgeführt wird.

8. Verfahren nach Anspruch 1, worin ein Trockenmittel zur Entfernung von Wasser nicht verwendet wird.

9. Verfahren nach Anspruch 1, worin R1 und R2 unabhängig C₁-C₈-Alkyl, C₃-C₈-Cycloalkyl sind, wobei jedes unabhängig substituiert ist mit einem oder mehreren S-R6, worin jedes R6 unabhängig ausgewählt ist aus C₁-C₈-Alkyl.

10. Verfahren nach Anspruch 1, worin R3 H ist.

11. Verfahren nach Anspruch 1, worin R4 und R5 jeweils unabhängig ausgewählt werden aus C₁-C₈-Alkyl und C₃-C₈-Cycloalkyl, oder worin R4 und R5 zusammengenommen mit N einen 5- oder 6-gliedrigen gesättigten oder ungesättigten Ring bedeuten.

12. Verfahren nach Anspruch 1, worin das erste Gemisch 3-Methylsulfanyl-butyraldehyd umfasst und das zweite Gemisch Pyrrolidin umfasst.

13. Verfahren nach Anspruch 1, worin das Enamin 1-(3-Methylsulfanyl-but-1-enyl)-pyrrolidin ist.

14. Verfahren nach Anspruch 1, worin das erste Gemisch und das zweite Gemisch in der Reaktionszone gleichzeitig während ihrer Zugabe in Kontakt gebracht werden können.

15. Verfahren nach Anspruch 1, worin das Rückgewinnungsgemisch ein Aminabscheidungsmittel umfasst, worin das Aminabscheidungsmittel Natriumhydroxid ist oder worin das Rückgewinnungsgemisch ein Aminabscheidungsmittel umfasst, worin das Aminabscheidungsmittel Natriumhydroxid ist und worin das Natriumhydroxid in H₂O konzentriert ist, mit von etwa 25 bis etwa 50 Gewichtsprozent Natriumhydroxid, oder worin das Rückgewinnungsgemisch ein Aminabscheidungsmittel umfasst, worin das Aminabscheidungsmittel eine Salzlösung ist

## Revendications

1. Procédé comportant les étapes consistant à :
(A) mettre en contact, dans une zone de réaction, un premier mélange avec un deuxième mélange, étant entendu que :
(1) ledit premier mélange comprend un composé carbonylé, un aldéhyde ou une cétone, répondant à la formule suivante : 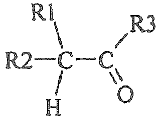 dans laquelle :
(a) R1 et R2 représentent chacun une entité choisie, indépendamment, dans l'ensemble constitué par les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle, chacun d'entre eux pouvant porter, indépendamment, un ou plusieurs substituant(s) symbolisé(s) par la formule S-R6 où chaque symbole R6 représente une entité choisie, indépendamment, dans l'ensemble constitué par les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle,
(b) et R3 représente une entité choisie dans l'ensemble constitué par l'atome d'hydrogène et les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle,
ou bien ledit composé carbonylé est du 3-méthylsulfanyl-butyraldéhyde,
(2) et ledit deuxième mélange comprend un solvant non polaire à point d'ébullition élevé et une amine répondant à la formule suivante : 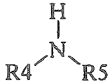 dans laquelle R4 et R5 représentent chacun une entité choisie, indépendamment, dans l'ensemble constitué par les groupes alkyle en C₁-C₈, cycloalkyle en C₃-C₈, alcoxy-alkyle en C₂-C₈, aryl-alkyle en C₇-C₁₂, alkyl-amino-alkyle en C₂-C₈, aryle et hétéroaryle, ou bien les entités représentées par R4 et R5 forment ensemble, avec l'atome d'azote, un cycle saturé ou insaturé à 5 ou 6 chaînons ;
(B) faire réagir, dans ladite zone de réaction, ladite amine et ledit composé carbonylé en vue de produire une énamine et de l'eau, étant entendu que la réaction est réalisée dans des conditions de distillation comprenant :
(1) une pression valant d'environ 100 pascals (Pa) à environ 120 000 Pa,
(2) et une température valant moins d'environ la valeur de la température de décomposition thermique de ladite énamine au cours de ladite réaction, mais de préférence, moins de cette valeur ;
(C) extraire une phase vapeur comprenant ledit solvant non polaire à point d'ébullition élevé, de l'amine et de l'eau ;
(D) faire condenser ladite phase vapeur extraite lors de l'étape (C), de manière à obtenir un condensat ;
(E) mettre ledit condensat obtenu à l'étape (D) en contact avec un mélange de récupération comprenant de l'eau et un agent de rejet d'amine, choisi dans l'ensemble formé par l'hydroxyde de sodium et une solution hypersaline, de manière à obtenir un mélange séparé comprenant ladite amine ;
(F) et, en option, renvoyer ladite amine récupérée à l'étape (E) dans ladite zone de réaction.

2. Procédé selon la revendication 1, dans lequel on utilise des quantités à peu près équimolaires de ladite amine et dudit composé carbonylé.

3. Procédé selon la revendication 1, dans lequel le rapport molaire de l'amine au composé carbonylé vaut d'environ 0,9 à environ 1,2, ou bien le rapport molaire de l'amine au composé carbonylé est supérieur à 1 mais inférieur à environ 1,1.

4. Procédé selon la revendication 1, dans lequel ledit solvant non polaire à point d'ébullition élevé est un solvant du type hydrocarbure aromatique, ou ledit solvant non polaire à point d'ébullition élevé est du benzène, ou bien ledit solvant non polaire à point d'ébullition élevé est du toluène, ou bien encore ledit solvant non polaire à point d'ébullition élevé est du xylène.

5. Procédé selon la revendication 1, dans lequel ladite réaction est réalisée dans des conditions de distillation qui comprennent une pression valant d'environ 1 000 Pa à environ 60 000 Pa et une température valant d'environ 10°C à environ 80°C, ou bien ladite réaction est réalisée dans des conditions de distillation qui comprennent une pression valant d'environ 2 500 Pa à environ 30 000 Pa et une température valant d'environ 20°C à environ 70°C, ou bien encore ladite réaction est réalisée dans des conditions de distillation qui comprennent une pression valant d'environ 5 000 Pa à environ 15 000 Pa et une température valant d'environ 25°C à environ 65°C.

6. Procédé selon la revendication 1, qui consiste à produire de la 1-(3-méthylsulfanyl-but-1-ényl)-pyrrolidine à une température valant moins d'environ la valeur de la température de décomposition thermique de ladite 1-(3-méthylsulfanyl-but-1-ényl)-pyrrolidine au cours de ladite réaction.

7. Procédé selon la revendication 1, dans lequel ladite réaction de condensation est effectuée dans des conditions azéotropiques.

8. Procédé selon la revendication 1, dans lequel il n'est pas fait usage d'un agent de dessiccation pour éliminer l'eau.

9. Procédé selon la revendication 1, dans lequel R1 et R2 représentent, indépendamment, un groupe alkyle en C₁-C₈ ou un groupe cycloalkyle en C₃-C₈, chacun d'entre eux pouvant porter, indépendamment, un ou plusieurs substituant(s) symbolisé(s) par la formule S-R6 où chaque symbole R6 représente une entité choisie, indépendamment, parmi les groupes alkyle en C₁-C₈.

10. Procédé selon la revendication 1, dans lequel R3 représente un atome d'hydrogène.

11. Procédé selon la revendication 1, dans lequel R4 et R5 représentent chacun une entité choisie, indépendamment, parmi les groupes alkyle en C₁-C₈ et cycloalkyle en C₃-C₈, ou bien les entités représentées par R4 et R5 forment ensemble, avec l'atome d'azote, un cycle saturé ou insaturé à 5 ou 6 chaînons.

12. Procédé selon la revendication 1, dans lequel ledit premier mélange comprend du 3-méthylsulfanyl-butyraldéhyde et ledit deuxième mélange comprend de la pyrrolidine.

13. Procédé selon la revendication 1, dans lequel ladite énamine est de la 1-(3-méthylsulfanyl-but-1-ényl)-pyrrolidine.

14. Procédé selon la revendication 1, dans lequel le premier mélange et le deuxième mélange peuvent être mis en contact dans la zone de réaction en même temps qu'ils sont introduits.

15. Procédé selon la revendication 1, dans lequel ledit mélange de récupération comprend un agent de rejet d'amine et ledit agent de rejet d'amine est de l'hydroxyde de sodium, ou bien ledit mélange de récupération comprend un agent de rejet d'amine et ledit agent de rejet d'amine est de l'hydroxyde de sodium concentré dans l'eau à raison d'environ 25 % à environ 50 % en poids d'hydroxyde de sodium, ou bien encore ledit mélange de récupération comprend un agent de rejet d'amine et ledit agent de rejet d'amine est une solution hypersaline.
